(19) Europäisches Patentamt
European Patent Office
Office européen des brevets

(11) Veröffentlichungsnummer : **0 035 754**
**B1**

(12) # EUROPÄISCHE PATENTSCHRIFT

(45) Veröffentlichungstag der Patentschrift : **24.08.83**

(51) Int. Cl.³ : **C 07 C 17/18**, C 07 C 69/743, C 07 C 67/307

(21) Anmeldenummer : **81101576.7**

(22) Anmeldetag : **05.03.81**

(54) **Verfahren zur Herstellung von 1,1-Dichlor-alkenen.**

(30) Priorität : **12.03.80 DE 3009485**

(43) Veröffentlichungstag der Anmeldung : **16.09.81 Patentblatt 81/37**

(45) Bekanntmachung des Hinweises auf die Patenterteilung : **24.08.83 Patentblatt 83/34**

(84) Benannte Vertragsstaaten : **AT BE CH DE FR GB IT LI NL**

(56) Entgegenhaltungen : **EP A 0 002 849**

(73) Patentinhaber : **BAYER AG**
**Zentralbereich Patente, Marken und Lizenzen**
**D-5090 Leverkusen 1, Bayerwerk (DE)**

(72) Erfinder : **Maurer, Fritz, Dr.**
**Roeberstrasse 8**
**D-5600 Wuppertal 1 (DE)**
Erfinder : **Priesnitz, Uwe, Dr.**
**Severinstrasse 58**
**D-5650 Solingen 1 (DE)**
Erfinder : **Riebel, Hans-Jochem, Dr.**
**In der Beek 92**
**D-5600 Wuppertal 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

# Verfahren zur Herstellung von 1,1-Dichlor-alkenen

Die Erfindung betrifft ein neues Verfahren zur Herstellung von 1,1-Dichloralkenen.

Es ist bekannt, daß man 1,1-Dichlor-alkene erhält, wenn man Lithiumsalze von Dichlormethanphosphonsäureestern mit Aldehyden oder Ketonen umsetzt (vergleiche Synthesis *1975,* 458-461 und 535-536). Die Herstellung der Lithiumsalze von Dichlormethanphosphonsäureestern ist jedoch aufwendig. Man erhält sie aus Chlormethanphosphonsäureestern bzw. Trichlormethanphosphonsäureestern durch Umsetzung mit Butyllithium und gegebenenfalls Tetrachlorkohlenstoff bei – 70 bis – 80 °C, wobei sorgfältig getrocknete Lösungsmittel zu verwenden sind und eine Inertgasatmosphäre erforderlich ist.

Es wurde nun ein Verfahren zur Herstellung von 1,1-Dichlor-alkenen der Formel I

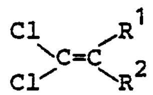 (I)

gefunden, in welcher

$R^1$ für Wasserstoff oder für einen gegebenenfalls substituierten Alkyl-, Alkenyl, Alkinyl, Cycloalkyl, Aralkyl, Aralkenyl oder Aryl-Rest steht und

$R^2$ für einen gegebenenfalls substituierten Alkyl-, Alkenyl-, Alkinyl-, Cycloalkyl-, Aralkyl-, Aralkenyl- oder Aryl-Rest steht, oder in welcher die beiden Reste $R^1$ und $R^2$ zusammen für eine gegebenenfalls verzweigte und/oder gegebenenfalls benzannellierte Kohlenwasserstoffkette stehen,

dadurch gekennzeichnet, daß man Carbonylverbindungen der Formel II

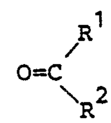 (II)

in welcher

$R^1$ und $R^2$ die oben angegebene Bedeutung haben, mit Trichlormethanphosphonsäureestern der Formel III

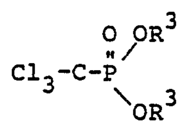 (III)

in welcher

$R^3$ einzeln für Alkyl oder Phenyl steht oder beide Reste $R^3$ zusammen für Alkandiyl (Alkylen) stehen, in Gegenwart von wenigstens der äquimolaren Menge Magnesium und gegebenenfalls in Gegenwart eines Verdünnungsmittels bei Temperaturen zwischen 0 und 150 °C umsetzt.

Es ist als überraschend anzusehen, daß man nach dem erfindungsgemäßen Verfahren 1,1-Dichloralkene der Formel (I) wesentlich einfacher und kostengünstiger in guten Ausbeuten erhält als man in Anbetracht des Standes der Technik erwarten konnte.

Verwendet man als Ausgangsstoffe beispielsweise 3-Formyl-2,2-dimethyl-cyclopropan-1-carbonsäuremethylester, Trichlormethanphosphonsäuredimethylester und Magnesium, so kann die Reaktion dieser Verbindungen durch folgendes Formelschema skizziert werden :

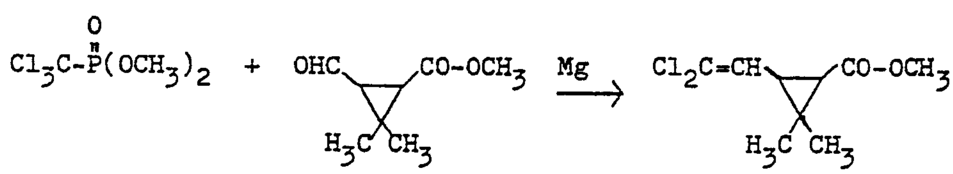


Die als Ausgangsstoffe zu verwendenden Carbonylverbindungen sind durch Formel (II) definiert.

Vorzugsweise stehen darin

$R^1$ für Wasserstoff, für gegebenenfalls halogen-substituiertes $C_1$-$C_5$-Alkyl, für gegebenenfalls halogensubstituiertes Benzyl oder Phenylethyl, oder für gegebenenfalls durch Halogen, $C_1$-$C_4$-Alkyl, $C_1$-$C_4$-Alkoxy, Methylendioxy, Cyano und/oder Nitro substituiertes Phenyl, und

$R^2$ für gegebenenfalls halogen-substituiertes $C_1$-$C_5$-Alkyl, für $C_2$-$C_5$-Alkenyl, für $C_2$-$C_5$-Alkinyl, für gegebenenfalls halogen-substituiertes Benzyl oder Phenylethyl, für gegebenenfalls durch Halogen, $C_1$-$C_4$-Alkyl, $C_1$-$C_4$-Alkoxy, Methylendioxy, Cyano und/oder Nitro substituiertes Phenyl, für gegebenenfalls

halogen-substituiertes Styryl oder für den Rest

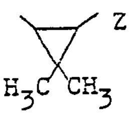

worin Z für Acetyl, Cyano, Carbamoyl, $C_1$-$C_4$-Alkoxycarbonyl oder für COOM steht, wobei M für Wasserstoff, ein Alkalimetall, ein Erdalkalimetalläquivalent oder einen Ammoniumrest steht.

Als Ausgangsstoffe besonders bevorzugt sind diejenigen Verbindungen der Formel (II), in welcher
$R^1$ für Wasserstoff steht und
$R^2$ für $C_2$-$C_5$-Alkenyl oder den Rest

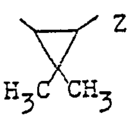

steht, worin
Z für Cyano, Acetyl, Carboxy, $C_1$-$C_4$-Alkoxycarbonyl oder für COOM steht, wobei M für Natrium oder Kalium steht.

Als Beispiele für die Ausgangsverbindungen der Formel (II) seien genannt :

β,β-Dimethyl-acrolein, 3-Formyl-2,2-dimethyl-1-cyanocyclopropan, 3-Formyl-2,2-dimethyl-1-acetylcyclopropan, 3-Formyl-2,2-dimethyl-cyclopropan-1-carbonsäure und deren Natriumsalz sowie 3-Formyl-2,2-dimethyl-cyclopropan-1-carbonsäure-methylester, -ethylester, -n-propylester, -iso-propylester, -n-butylester, -iso-butylester, -sek.-butylester und -tert.-butylester.

Die Verbindungen der Formel (II) sind teilweise bekannt (vergleiche Synthesis *1975,* 535-536 ; Tetrahedron Lett. *1976,* 1979-1982). Die besonders bevorzugten Cyclopropanderivate der Formel (II) sind teilweise noch nicht in der Literatur beschrieben. Man erhält diese Verbindungen nach an sich bekannten Verfahren. Ein Syntheseweg ist in nachstehendem Formelschema skizziert :
(R steht für $C_1$-$C_4$-Alkyl)

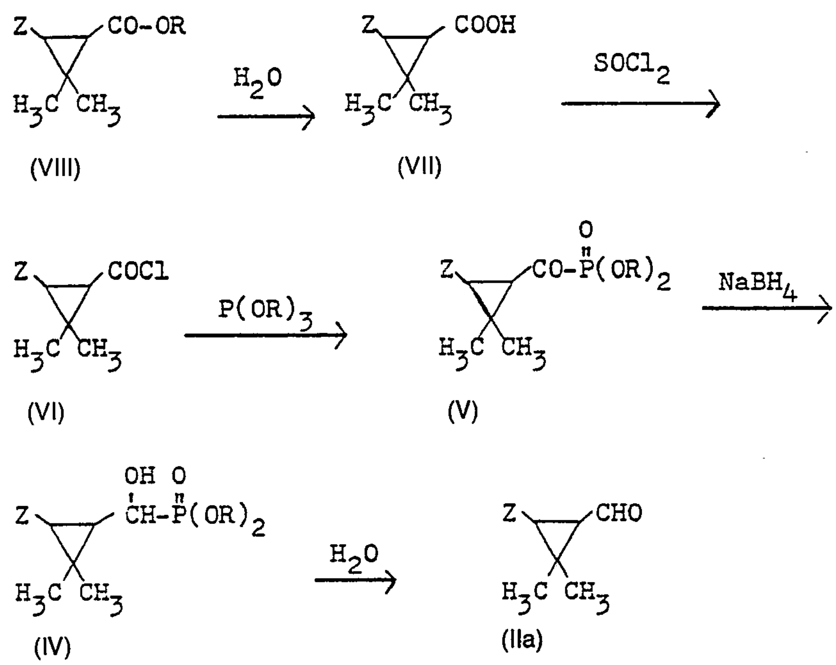


(VIII) (VII) (VI) (V) (IV) (IIa)

Durch Hydrolyse von bekannten Cyclopropancarbonsäureestern der Formel (VIII) (vergleiche J. Org. Chem. *32* (1967), 3351-3355 ; Bull. Soc. Chim. Belg. *87* (1978), 721-732 ; Tetrahedron Lett. *1978,* 1847-1850), beispielsweise durch Umsetzung mit wässrig-alkoholischer Kalilauge bei Temperaturen zwischen 20 und 100 °C und anschließendes Ansäuren erhält man die Carbonsäuren der Formel (VII). Diese können durch Umsetzung mit Halogenierungsmitteln, wie z. B. Thionylchlorid, bei Temperaturen zwischen 20 und 80 °C in die Säurechloride der Formel (VI) umgewandelt werden.

Durch Umsetzung der Säurechloride (VI) mit Trialkylphosphiten bei Temperaturen zwischen – 20 und

+ 150 °C, vorzugsweise zwischen 0 und 120 °C, erhält man die Cyclopropanoylphosphonsäureester der Formel (V) (vergleiche J. Am. Chem. Soc. *86* (1964), 3862-3866 ; Methoden der organischen Chemie (Houben-Weyl-Müller), 4. Auflage, Band 12/1, S. 453, Georg-Thieme-Verlag, Stuttgart 1963). Zur Isolierung und Reinigung der Produkte wird, gegebenenfalls unter vermindertem Druck destilliert.

Die α-Hydroxy-phosphonsäureester der Formel (IV) erhält man durch Reduktion der Oxoverbindungen der Formel (V) mit Natriumtetrahydridoborat, gegebenenfalls unter Verwendung eines Verdünnungsmittels, wie z. B. Wasser oder wässriges Methanol, bei Temperaturen zwischen − 20 und + 50 °C und Halten des pH-Wertes zwischen 5 und 8 durch Zugabe eines Puffermittels, wie z. B. Natriumhydrogenphosphat (vergleiche Chem. Ber. *103* (1970), 2984-2986). Zur Aufarbeitung extrahiert man mit einem mit Wasser nicht mischbaren Lösungsmittel, wie z. B. Methylenchlorid, trocknet die Extrakte, filtriert und destilliert das Lösungsmittel unter vermindertem Druck ab.

Aus den α-Hydroxy-phosphonsäureestern der Formel (IV) können die entsprechenden Aldehyde der Formel (IIa) durch Behandeln mit Natronlauge bei Temperaturen zwischen 0 und 100 °C, vorzugsweise zwischen 10 und 50 °C hergestellt werden (vergleiche Chem. Ber. *103* (1970), 2984-2986).

Alternativ zum oben skizzierten Herstellungsverfahren erhält man Aldehyde der Formel (IIa) auch durch Umsetzung von Säurechloriden der Formel (VI) mit Lithiumtritert.-butoxy-hydrido-aluminat, welches man gegebenenfalls in situ aus Lithium-tetrahydridoaluminat und tert.-Butanol hergestellt hat, gegebenenfalls in Gegenwart eines Verdünnungsmittels, wie z. B. Tetrahydrofuran, bei Temperaturen zwischen − 100 und + 100 °C, vorzugsweise zwischen − 80 und + 50 °C. Zur Aufarbeitung wird in eine Mischung aus Salzsäure und Eiswasser gegossen und mit einem mit Wasser nicht mischbaren Lösungsmittel, wie z. B. Diethylether, extrahiert. Die Extrakte werden getrocknet, filtriert und eingeengt. Zur Reinigung des Rohproduktes wird gegebenenfalls destilliert.

Die weiter als Ausgangsstoffe zu verwendenden Trichlormethanphosphonsäureester sind durch Formel (III) definiert. Vorzugsweise stehen darin die Reste

$R^3$ einzeln für $C_1$-$C_4$-Alkyl oder Phenyl oder beide Reste $R^3$ stehen zusammen für $C_2$-$C_5$-Alkandiyl.

Als Beispiele seien Trichlormethanphosphonsäure-dimethylester, -diethylester, -dipropylester und -diphenylester genannt.

Verbindungen der Formel (III) sind bekannt (vergleiche J. Am. Chem. Soc. *69* (1947), 1002 ; ibid. *77* (1955), 1156).

Das erfindungsgemäße Verfahren wird vorzugsweise unter Verwendung von Verdünnungsmitteln durchgeführt. Als solche kommen praktisch alle inerten organischen Lösungsmittel insbesondere aprotisch polare Solventien in Frage.

Hierzu gehören Ether, wie z. B. Glycoldimethylether, Diglycoldimethylether, Tetrahydrofuran und Dioxan, Carbonsäureamide, wie z. B. Dimethylformamid, Dimethylacetamid und N-Methyl-pyrrolidon, Sulfoxide, wie z. B. Dimethylsulfoxid, Phosphorsäureamide, wie z. B. Hexamethylphosphorsäuretriamid, sowie Nitrile, wie z. B. Acetonitril und Propionitril.

Die Reaktionstemperatur kann innerhalb eines größeren Bereichs variiert werden. Im allgemeinen arbeitet man zwischen 0 und 150 °C, vorzugsweise bei 10 bis 80 °C.

Das erfindungsgemäße Verfahren wird im allgemeinen bei Normaldruck durchgeführt.

Je Mol Carbonylverbindung der Formel (II) werden 0,9 bis 1,2 Mol, vorzugsweise 0,95 bis 1,1 Mol Trichlormethanphosphonsäureester der Formel (III) und 1 bis 5 Mol, vorzugsweise 1,5 bis 4 Mol Magnesium eingesetzt.

Zur Durchführung des erfindungsgemäßen Verfahrens wird vorzugsweise das Magnesium in einem Verdünnungsmittel vorgelegt und die Ausgangsstoffe der Formeln (II) und (III) werden gleichzeitig zugetropft. Das Reaktionsgemisch wird bis zum Ende der Umsetzung gerührt und dann filtriert.

Die Aufarbeitung kann nach üblichen Methoden durchgeführt werden, beispielsweise, indem man das Filtrat mit Wasser verdünnt, mit einem mit Wasser praktisch nicht mischbaren, organischen Lösungsmittel, wie z. B. Methylenchlorid, extrahiert, die organische Phase trocknet, filtriert und eindampft. Die im Rückstand verbleibenden Produkte können auf übliche Weise, z. B. durch Vakuumdestillation, gereinigt werden.

Die nach dem erfindungsgemäßen Verfahren herzustellenden 1,1-Dichlor-alkene können zum Teil als Zwischenprodukte zur Herstellung von insektizid und akarizid wirksamen Pyrethroiden verwendet werden (vergleiche DE-OS 2 326 077).

## Herstellungsbeispiele

### Beispiel 1

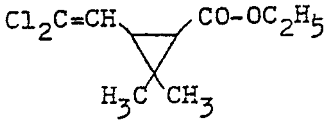

Zu 9,6 g aktivierten Magnesiumspänen in 100 ml Dimethylformamid wird ein Gemisch aus 25,5 g (0,1 Mol) Trichlormethanphosphonsäurediethylester und 17,2 g (0,1 Mol) 3-Formyl-2,2-dimethyl-cyclopro-

pan-1-carbonsäureethylester in der Weise getropft, daß die Reaktionstemperatur 60 °C nicht übersteigt. Nach Ende der exothermen Reaktion wird auf Raumtemperatur abkühlen gelassen. Das Reaktionsgemisch wird dann filtriert. Das Filtrat wird mit 200 ml Wasser versetzt; diese Lösung wird 2 mal mit je 100 ml Methylenchlorid extrahiert. Die vereinigten Methylenchloridextrakte werden über Natriumsulfat getrocknet und filtriert. Das Filtrat wird destilliert. Man erhält 3-(2,2-Dichlor-vinyl)-2,2-dimethyl-cyclopropan-1-carbonsäureethylester in einer Ausbeute von 60 % der Theorie.

Der Strukturbeweis wurde durch vergleichende Gaschromatographie und durch Kernresonanzspektroskopie geführt.

Beispiel 2

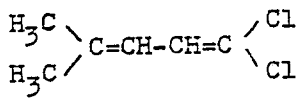

2,4 g aktivierte Magnesiumspäne werden in 50 ml Dimethylformamid angeschlämmt und mit einer Mischung aus 12,8 g (0,05 Mol) Trichlormethanphosphonsäurediethylester und 4,2 g (0,05 Mol) β,β-Dimethylacrolein versetzt. Die Temperatur steigt bis ca. 45 °C an. Man rührt das Gemisch 1 Stunde bei 60-70 °C, gibt 300 ml Wasser zu und extrahiert 3 mal mit je 50 ml n-Hexan. Die organischen Auszüge werden über Natriumsulfat getrocknet und im Vakuum eingedampft. Den Rückstand destilliert man im Vakuum. Man erhält so 5,2 g (69 % der Theorie) 1,1-Dichlor-3,3-dimethylbutadien in Form einer farblosen Flüssigkeit mit dem Siedepunkt 53-55 °C/10 Torr.

**Anspruch**

Verfahren zur Herstellung von 1,1-Dichlor-alkenen der Formel I

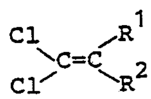

(I)

in welcher

$R^1$ für Wasserstoff oder für einen gegebenenfalls substituierten Alkyl-, Alkenyl, Alkinyl, Cycloalkyl, Aralkyl, Aralkenyl oder Aryl-Rest steht und

$R^2$ für einen gegebenenfalls substituierten Alkyl-, Alkenyl-, Alkinyl-, Cycloalkyl-, Aralkyl-, Aralkenyl- oder Aryl-Rest steht, oder in welcher die beiden Reste $R^1$ und $R^2$ zusammen für eine gegebenenfalls verzweigte und/oder gegebenenfalls benzannellierte Kohlenwasserstoffkette stehen,

dadurch gekennzeichnet, daß man Carbonylverbindungen der Formel II

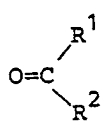

(II)

in welcher

$R^1$ und $R^2$ die oben angegebene Bedeutung haben, mit Trichlormethanphosphonsäureestern der Formel III

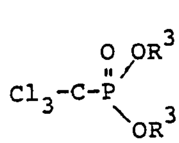

(III)

in welcher

$R^3$ einzeln für Alkyl oder Phenyl steht oder beide Reste $R^3$ zusammen für Alkandiyl (Alkylen) stehen, in Gegenwart von wenigstens der äquimolaren Menge Magnesium und gegebenenfalls in Gegenwart eines Verdünnungsmittels bei Temperaturen zwischen 0 und 150 °C umsetzt.

**Claim**

Process for the preparation of 1,1-dichloroalkenes of the formula I

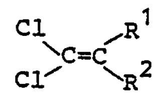

(I)

in which

$R^1$ represents hydrogen or an optionally substituted alkyl, alkenyl, alkinyl, cycloalkyl, aralkyl, aralkenyl or aryl radical and

$R^2$ represents an optionally substituted alkyl, alkenyl, alkinyl, cycloalkyl, aralkyl, aralkenyl or aryl radical, or in which the two radicals $R^1$ and $R^2$ together represent an optionally branched and/or optionally benzofused hydrocarbon chain,

characterised in that carbonyl compounds of the formula II

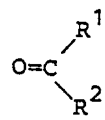

(II)

in which

$R^1$ and $R^2$ have the abovementioned meaning, are reacted with trichloromethanephosphonic acid esters of the formula III

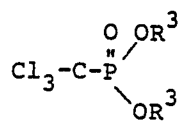

(III)

in which

the two radicals $R^3$ individually represent alkyl or phenyl or the two radicals $R^3$ together represent alkanediyl (alkylene), in the presence of at least the equimolar amount of magnesium and if appropriate in the presence of a diluent, at temperatures between 0 and 150 °C.

**Revendication**

Procédé de fabrication de 1,1-dichloroalcènes de formule I

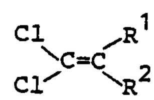

(I)

dans laquelle

$R^1$ représente de l'hydrogène ou un radical alcoyle, alcényle, alcinyle, cycloalcoyle, aralcoyle, aralcényle ou aryle éventuellement substitué et

$R^2$ représente un radical alcoyle, alcényle, alcinyle, cycloalcoyle, aralcoyle, aralcényle ou aryle éventuellement substitué, ou dans laquelle les deux radicaux $R^1$ et $R^2$ représentent ensemble une chaîne hydrocarbonée éventuellement ramifiée et/ou éventuellement fusionnée à un noyau benzénique, caractérisé en ce qu'on fait réagir des composés carbonylés de formule II

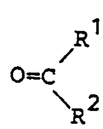

(II)

dans laquelle

$R^1$ et $R^2$ ont la signification indiquée plus haut, avec des esters d'acide trichlorométhane-phosphonique de formule III

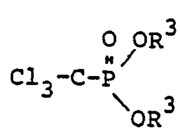

(III)

dans laquelle les

$R^3$ représentent individuellement un alcoyle ou phényle, ou bien les deux radicaux $R^3$ représentent ensemble un alcane-diyle (alcoylène), en présence d'au moins la quantité équimolaire de magnésium et éventuellement en présence d'un diluant à des températures entre 0 et 150 °C.